# EUROPEAN PATENT SPECIFICATION

(11) **EP 1 581 617 B1**
(45) Date of publication and mention of the grant of the patent: **02.02.2011**
(21) Application number: 03707738.5
(22) Date of filing: 06.02.2003
(51) Int. Cl.: C12P 7/06, C12P 7/10, C12P 7/18, C12P 7/20, C12P 7/44, C12P 7/56, C12P 7/58, C12P 7/60, C12P 19/02

(54) **METHODS FOR PRODUCING END-PRODUCTS FROM CARBON SUBSTRATES**
VERFAHREN ZUR HERSTELLUNG VON ENDPRODUKTEN AUS KOHLENSTOFFSUBSTRATEN
PROCEDES DE PRODUCTION DE PRODUITS FINAUX A PARTIR DE SUBSTRATS DE CARBONE

(30) Priority: 08.02.2002 US 355260 P
(43) Date of publication of application: 05.10.2005
(73) Proprietor: GENENCOR INTERNATIONAL, INC., Palo Alto, California 94304 (US)
(72) Inventor: CHOTANI, Gopal, K., Cupertino, CA 95014 (US); KUMAR, Manoj, Fremont, CA 94555 (US); PUCCI, Jeff, P., Pacifica, CA 94044 (US); SANFORD, Karl, J., Cupertino, CA 95014 (US); SHETTY, Jayarama, K., Pleasanton, CA 94566 (US)
(74) Representative: Forrest, Graham Robert
(86) International application number: PCT/US2003/003532
(87) International publication number: WO 2003/066816

(56) References cited:
- WO-A-93/25714
- WO-A2-02/00858
- US-A- 4 316 956
- US-A- 4 514 496
- US-A- 4 945 052
- US-A- 5 152 969
- US-A- 5 792 631
- KUMAGAI M H ET AL: "CONVERSION OF STARCH TO ETHANOL IN A RECOMBINANT SACCHAROMYCES CEREVISIAE STRAIN EXPRESSING RICE ALPHA-AMYLASE FROM A NOVEL PICHIA PASTORIS ALCOHOL OXIDASE PROMOTER" BIO/TECHNOLOGY, NATURE PUBLISHING CO. NEW YORK, US, vol. 11, May 1993 (1993-05), pages 606-610, XP002037995 ISSN: 0733-222X
- MUJOO RAJNI ET AL: "Susceptibility of starch to in vitro enzyme hydrolysis in rice, rice flakes and intermediary products" LEBENSMITTEL-WISSENSCHAFT AND TECHNOLOGIE, vol. 31, no. 2, 1998, pages 114-121, XP002403948 ISSN: 0023-6438
- ZHOU SHENGDE ET AL: "Gene integration and expression and extracellular secretion of Erwinia chrysanthemi endoglucanase CelY (celY) and CelZ (celZ) in ethanologenic Klebsiella oxytoca P2" APPLIED AND ENVIRONMENTAL MICROBIOLOGY, WASHINGTON,DC, US, vol. 67, no. 1, January 2001 (2001-01), pages 6-14, XP002192787 ISSN: 0099-2240
- JANSE B J H ET AL: "One-step enzymatic hydrolysis of starch using a recombinant strain of saccharomyces cerevisiae producing alpha-amylase, glucoamylase and pullulanase" APPLIED MICROBIOLOGY AND BIOTECHNOLOGY, SPRINGER VERLAG, BERLIN, DE, vol. 42, 1995, pages 878-883, XP002958907 ISSN: 0175-7598
- SAHA ET AL: 'Alcoholic Fermentation of Raw Sweet Potato by a Nonconventional Method Using Endomycopsis fibulegera Glucoamylase Preparation' BIOTECHNOLOGY AND BIOENGINEERING vol. 25, no. 4, 1983, pages 1181 - 1186, XP003007935
- UEDA ET AL: 'Production of Ethanol from Raw Cassava Starch by a Nonconventional Fermentation Methods' BIOTECHNOLOGY AND BIOENGINEERING vol. 23, no. 2, 1981, pages 291 - 299, XP002380368
- UEDA ET AL: 'Alcoholic Fermentation of Raw Starch without Cooking by using Black-koji Amylase' J FERMENT TECHNOL. vol. 58, no. 3, 1980, pages 237 - 242, XP009062486

## Description

### FIELD OF THE INVENTION

The present invention provides means for the production of lactic and from granular starch. The methods of the present invention do not require gelatinization or liquefaction of the substrate.

### BACKGROUND OF THE INVENTION

Industrial fermentations predominantly use glucose as feed-stock for the production of proteins, enzymes and chemicals. These fermentations are usually batch, fed-batch, or continuous, and operate under substrate-limited and minimal by-products forming conditions. These are critical operating conditions that must be controlled during fermentation in order to optimize fermentation time, yield and efficiency. Currently used methods and feed-stocks have drawbacks that reduce the efficiency of the fermentation processes.

Glucose is a natural, carbon based compound that is useful in a multitude of chemical and biological synthetic applications as a starting substrate. However, syrups that contain glucose purity levels of greater than 90% are relatively expensive. In addition, the presence of high glucose concentrations increases the susceptibility of the fermentation system to microbial contamination, thereby resulting in an adverse effect upon the production efficiency. Another disadvantage is that even the presence of low to moderate levels of glucose in the fermentation vat adversely affects the conversion of the glucose to the desired end product, for example by enzymatic inhibition and/or catabolite repression, and/or the growth of microorganisms. As a result, various attempts have been made to reduce the costs of industrial fermentation, particularly in utilization of less expensive substrates than glucose. However, despite the development of numerous approaches, there remains a need in the art for economical, efficiently-utilized substrates for fermentation. Indeed, there is a great need in the art for methods that utilize a less expensive starting material than glucose to more efficiently produce a desired end-product.

### SUMMARY OF THE INVENTION

The invention provides a method for producing lactic acid comprising:
contacting a slurry comprising granular starch with a glucoamylase which is capable of hydrolyzing both the linear and branched glucosidic linkages of starch, an alpha-amylase, and a lactate-producing *Lactobacillus,* in a simultaneous saccharification and fermentation process;
wherein said starch is not subjected to liquefaction or gelatinization.

The granular starch may be derived from wheat, barley, sweet potato, tapioca, corn, maize, cassava, milo, rye, bran or rice.

The alpha-amylase may be from a *Bacillus* species, e.g. from *Bacillus licheniformis,* or may be from a different organism such as *Aspergillus oryzae.*

The glucoamylase may be from a *Humicola* or *Rhizopus* species.

The *Lactobacillus* may be *Lactobacillus amylovorous.*

In certain embodiments, both the glucoamylase and alpha-amylase may be from *Rhizopus.*

The slurry comprising the granular starch may be subject to 65°C pasteurization before contacting with the starch hydrolyzing enzyme.

The pH of the medium may be pH 5.0 to 9.0.

The fermentation may take place at a temperature range of 25°C to 35°C.

The granular starch may be present in the slurry in a concentration of 10% to 35% w/v.

The method may further comprise the step of recovering the lactic acid produced.

### DESCRIPTION OF THE FIGURES

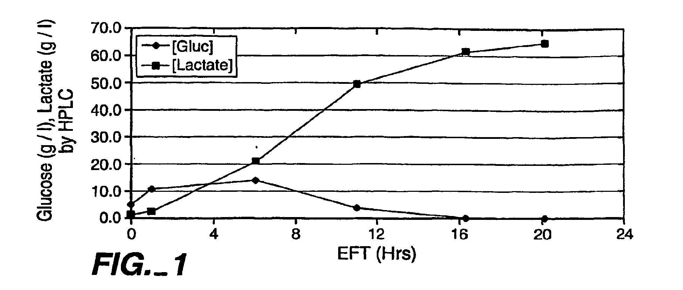

Figure 1 provides a graph showing the bioconversion of granular starch to glucose and lactate.

### DESCRIPTION OF THE INVENTION

There is a great need in the art for methods in which less expensive starting materials than glucose are used to efficiently produce lactic acid As described in greater detail herein, the present invention provides methods using starch (*e.g*., com and wheat starch) as a substrate.

Starch is a plant-based fermentation carbon source. Com starch and wheat starch are carbon sources that are much cheaper than glucose carbon feedstock for fermentation. Conversion of liquefied starch to glucose is known in the art and is generally carried out using enzymes such alpha-amylase, pullulanase, and glucoamylase. A large number of processes have been described for converting liquefied starch to the monosaccharide, glucose. Glucose has value in itself, and also as a precursor for other saccharides such as fructose. In addition, glucose may also be fermented to ethanol or other fermentation products. However the ability of the enzymatic conversion of a first carbon source to the intermediate, especially glucose, may be impaired by the presence of the intermediate.

For example, the typical methods used in Japanese sake brewing and alcoholic production use starch without cooking. However, these techniques require some special operations such as acidification of mash (pH 3.5), which prevents contamination of harmful microorganisms. Furthermore, these methods require a longer period of the time for the saccharification and fermentation than the present invention. In addition, these methods require complex process steps such as dialysis of a fermented broth and are too cumbersome to utilize in the general production of products via fermentation.

The use of soluble dextrins and glucose as feed-stock in fermentations have various drawbacks, including high processing cost, and problems associated with viscosity and oxygen transfer. In addition, in comparison to the present invention, these methods produce lower yields of the desired products and more problems associated with the formation of by-products. Indeed, the costs of converting starch or biomass to dextrins are substantial and involve high energy input, separate reactor tanks, more time, a detailed bioprocess operation, incomplete saccharification, back-reaction, and enzymes associated with the typical pre-fermentation saccharification step. These problems have led to a number of attempts to provide methods for conversion directly to starch within one reaction vessel or container and at lower temperatures. Biotransformation of a carbohydrate source to 1,3-propanediol in mixed cultures is described in US Pat. No. 5,599,689 (Haynie, *et al*.). The method described by Haynie *et al.,* involves mixing a glycerol (*i.e*., an intermediate) producing organism with a diol producing organism (*i.e*., an end-product), contacting the mixed culture medium with a carbon substrate and incubating the mixed culture medium to produce the desired end-product, 1,3-propanedlol. In U.S. Patent No. 4,514,496, Yoshizuma describes methods that involve maintaining the concentration of the raw material in the scurry relative the mashing liquid to produce alcohol by fermentation without cooking (*i.e*., without high temperature liquefaction before saccharization. Nonetheless, these methods lack the efficiency and economical advantages provided by the present invention.

### Definitions

Unless defined otherwise herein, all technical and scientific terms used herein have the same meaning as commonly understood by one of ordinary skill in the art to which this invention belongs. Various references (*See e.g.*, Singleton, et al., DICTIONARY OF MICROBIOLOGY AND MOLECULAR BIOLOGY, 2D ED., John Wiley and Sons, New York [1994]; and Hale and Marham, THE HARPER COLLINS DICTIONARY OF BIOLOGY, Harper Perennial, NY [1991]) provide general definitions of many of the terms used herein.

Although any methods and materials similar or equivalent to those described herein find use in the practice of the present invention, preferred methods and materials are described herein. Numeric ranges are inclusive of the numbers defining the range. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary.

The headings provided herein are not limitations of the various aspects or embodiments of the invention which can be had by reference to the specification as a whole. Furthermore, the terms defined immediately below are more fully defined by reference to the Specification as a whole.

As used herein, the term "carbon substrate" refers to a material containing at least one carbon atom which can be enzymatically converted into an intermediate for subsequent conversion into the desired carbon end-product. Exemplary carbon substrates include, but are not limited to biomass, starches, dextrins and sugars.

As used herein, "biomass" refers to cellulose- and/or starch-containing raw materials, including but not limited to wood chips, corn stover, rice, grasses, forages, perrie-grass, potatoes, tubers, roots, whole ground corn, cobs, grains, wheat, barley, rye, milo, brans, cereals, sugar-containing raw materials (*e.g*., molasses, fruit materials, sugar cane or sugar beets), wood, and plant residues. Indeed, it is not intended that the present invention be limited to any particular material used as biomass. In preferred embodiments of the present invention, the raw materials are starch-containing raw materials (*e.g*., cobs, whole ground corns, corns, grains, milo, and/or cereals, and mixtures thereof). In particularly preferred embodiments, the term refers to any starch-containing material originally obtained from any plant source.

As used herein, "starch" refers to any starch-containing materials. In particular, the term refers to various plant-based materials, including but not limited to wheat, barley, potato, sweet potato, tapioca, corn, maize, cassava, milo, rye, and brans. Indeed, it is not intended that the present invention be limited to any particular type and/or source of starch. In general, the term refers to any material comprised of the complex polysaccharide carbohydrates of plants, comprised of amylose and amylopectin, with the formula (C₆H₁₀O₅)*ₓ,* wherein "*x*" can be any number.

As used herein, "cellulose" refers to any cellulose-containing materials. In particular, the term refers to the polymer of glucose (or "cellobiose"), with the formula (C₆H₁₀O₅)*ₓ*, wherein "x" can be any number. Cellulose is the chief constituent of plant cell walls and Is among the most abundant organic substances in nature. While there is a β-glucoside linkage in cellulose, there is a an α-glucoside linkage in starch. In combination with lignin, cellulose forms "lignocellulose."

As used herein, "intermediate" refers to a compound that contains at least one carbon atom into which the carbon substrates are enzymatically converted. Exemplary intermediates include, but are not limited to pentoses (*e.g*., xylose, arabinose, lyxose, ribose, ribulose, xylulose); hexoses (*e.g*., glucose, allose, altrose, mannose, gulose, idose, galactose, talose, psicose, fructose, sorbose, and tagatose); and organic acids thereof.

As used herein, the term "enzymatic conversion" refers to the modification of a carbon substrate to an intermediate or the modification of an intermediate to an end-product by contacting the substrate or intermediate with an enzyme. In some embodiments, contact is made by directly exposing the substrate or intermediate to the appropriate enzyme. In other embodiments, contacting comprises exposing the substrate or intermediate to an organism that expresses and/or excretes the enzyme, and/or metabolizes the desired substrate and/or intermediate to the desired intermediate and/or end-product, respectively.

As used herein, the term "starch hydrolyzing enzyme " refers to any enzyme that is capable of converting starch to the intermediate sugar (*e.g*., a hexose or pentose).

As used herein, "monosaccharide" refers to any compound having an empirical formula of (CH₂O)ₙ, wherein n is 3-7, and preferably 5-7. In some embodiments, the term refers to "simple sugars" that consist of a single polyhydroxy aldehyde or ketone unit. The term encompasses, but is not limited to such compounds as glucose, galactose, and fructose.

As used herein, "disaccharide" refers to any compound that comprises two covalently linked monosaccharide units. The term encompasses, but is not limited to such compounds as sucrose, lactose and maltose.

As used herein, "oligosaccharide" refers to any compound having 2 - 10 monosaccharide units joined in glycosidic linkages. In some preferred embodiments, the term refers to short chains of monosaccharide units joined together by covalent bonds.

As used herein, "polysaccharide" refers to any compound having multiple monosaccharide units joined in a linear or branched chain. In some preferred embodiments, the term refers to long chains with hundreds or thousands of monosaccharide units. Some polysaccharides, such as cellulose have linear chains, while others (*e.g*., glycogen) have branched chains. Among the most abundant polysaccharides are starch and cellulose, which consist of recurring glucose units (although these compounds differ in how the glucose units are linked).

As used herein, "culturing" refers to fermentative bioconversion of a carbon substrate to the desired end-product (typically within a reaction vessel). In particularly preferred embodiments, culturing involves the growth of microorganisms under suitable conditions for the production of the desired end-product(s).

As used herein, the term "saccharification" refers to converting a directly unusable polysaccharide to a useful sugar feed-stock for bioconversion or fermentative bioconversion.

As used herein, the term "fermentation" refers to the enzymatic and anaerobic breakdown of organic substances by microorganisms to produce simpler organic products. In preferred embodiments, fermentation refers to the utilization of carbohydrates by microorganisms (*e.g*., bacteria) involving an oxidation-reduction metabolic process that takes place under anaerobic conditions and in which an organic substrate serves as the final hydrogen acceptor (*i.e*., rather than oxygen). Although fermentation occurs under anaerobic conditions, it is not intended that the term be solely limited to strict anaerobic conditions, as fermentation also occurs in the presence of oxygen.

As used herein, the terms "substantially all consumed" and "substantially all bioconverted" refer to the maintenance of a low level of intermediate in a conversion medium which adversely affects the enzymatic inhibition, oxygen transfer, yield, by-product minimization and/or catabolite repression effects of the intermediate (*e.g*., a hexose), upon the ability of the intermediate converting enzyme to convert the intermediate to the end-product or another intermediate and/or the ability of the substrate converting enzyme to convert the substrate to the intermediate.

As used herein, the terms "bioconversion" and "bioconverted" refer to contacting a microorganism with the carbon substrate or intermediate, under conditions such that the carbon substrate or intermediate is converted to the intermediate or desired end-product, respectively. In some embodiments, these terms are used to describe the production of another intervening intermediate in *in vitro* methods in which biocatalysts alone are used. In some preferred embodiments, the terms encompass metabolism by microorganisms and/or expression or secretion of enzyme(s) that achieve the desired conversion.

As used herein, the terms "conversion media" and "conversion medium" refer to the medium/media in which the enzymes and the carbon substrate, intermediate and end-products are in contact with one another. These terms include, but are not limited to fermentation media, organic and/or aqueous media dissolving or otherwise suspending the enzymes and the carbon substrate, intermediate and end-products. In some embodiments, the media are complex, while in other preferred embodiments, the media are defined.

As used herein, the term "end-product" refers to any carbon-source derived molecule product which is enzymatically converted from the intermediate. In particularly preferred embodiments, the methods of the present invention are used in order to produce a "desired end-product" (*i.e*., the product that is intended to be produced through the use of these methods).

As used herein, "low concentration" refers to a concentration level of a compound that is less than that would result in the production of detrimental effects due to the presence of the compound. In particularly preferred embodiments, the term is used in reference to the concentration of a particular intermediate below which the detrimental effects of catabolite suppression and/or enzyme inhibition are observed. In some embodiments, the term refers to the concentration level of a particular intermediate above which triggers catabolite repression and/or enzymes inhibition by substrate and/or products.

As used herein, the phrase "maintained at a level below which triggers catabolite repression effects" refers to maintaining the concentration of an intermediate to below that level which triggers catabolite repression.

As used herein, the term "reduces catabolite repression" means conditions under which the effects of catabolite repression are produced. In preferred embodiments, the term refers to conditions in which the intermediate concentration is less than that threshold which triggers catabolite repressive effects.

As used herein, the term "reduces enzyme inhibition" means conditions under which the inhibition of an enzyme is reduced as compared to the inhibition of the enzyme under usual, standard conditions. In preferred embodiments of the present invention, the term refers to conditions in which the concentration of an intermediate, substrate and/or product of the enzyme reaction is less than that threshold which triggers enzyme inhibition.

As used herein, the term "substrate converting enzyme" refers to any enzyme that converts the substrate (*e.g*., granular starch) to an intermediate, (*e.g*., glucose). Substrate converting enzymes include, but are not limited to alpha-amylases, glucoamylases, pullulanases, starch hydrolyzing enzymes and various combinations thereof.

As used herein, the term "intermediate converting enzyme" refers to any enzyme that converts an intermediate (*e.g*., D-glucose, D-fructose, etc.), to the desired end-product. In preferred embodiments, this conversion is accomplished through hydrolysis, while in other embodiments, the conversion involves the metabolism of the intermediate to the end-product by a microorganism. However, it is not intended that the present invention be limited to any particular enzyme or means of conversion. Indeed, it is intended that any appropriate enzyme will find use in the various embodiments of the present invention.

As used herein, "yield" refers to the amount of end-product or intermediate produced using the methods of the present invention. In some preferred embodiments, the yield produced using the methods of the present invention is greater than that produced using methods known in the art. In some embodiments, the yield refers to the volume of the end-product or intermediate, while in other embodiments, the term is used in reference to the concentration of the end-product or intermediate in a composition.

As used herein, the term "oxygen transfer" refers to having sufficient dissolved oxygen in the bioconversion and/or fermentative bioconversion medium transferred form gas phase to a liquid medium such that it is not a rate limiting step.

As used herein, "by-product formation" refers to the production of products that are not desired. In some preferred embodiments, the present invention provides methods that avoid or reduce the production of by-products, as compared to methods known in the art.

As used herein, the term "enzymatic inhibition" refers to loss of enzyme activity by either physical or biochemical effects on the enzyme. In some embodiments, inhibition results from the effects of the product formed by the enzyme activity, while in other embodiments, inhibition results from the action of the substrate or intermediate on the enzyme.

As used herein, "enzyme activity" refers to the action of an enzyme on its substrate. In some embodiments, the enzyme activity is quantitated using means to determine the conversion of the substrate to the intermediate, while in other embodiments, the conversion of the substrate to the end-product is determined, while in still further embodiments, the conversion of the intermediate to the end-product is determined.

As used herein, the term "enzyme unit" refers to the amount of enzyme which converts 1 micromole of substrate per minute to the substrate product at optimum assay conditions (unless otherwise noted). In some embodiments, commercially available enzymes (*e.g*., SPEZYME®, DISTALLASE®, OPTIMAX®; Genencor International) find use in the methods of the present invention.

As used herein, the term "glucoamylase unit" (GAU) is defined as the amount of enzyme required to produce one micromole of glucose per minute under assay conditions of 40° C. and pH 5.0.

As used herein, the term "glucose oxidase unit" (GOU) is defined as the amount of enzyme required to oxidize one micromole of D-glucose per minute under assay conditions of 25° C. and pH 7.0, to gluconic acid.

As used herein, the term "catalase units" (CU) is defined as the amount of enzyme required to decompose 1 micromole of hydrogen peroxide per minute under assay conditions of 25° C. and pH 7.0.

As used herein, one AG unit (GAU) is the amount of enzyme which splits one micromole of maltose per minute at 25° C. and pH 4.3. In some embodiments of the present invention, a commercially available liquid form of glucoamylase (OPTIDEX® L-400; Genencor International) with an activity of 400 GAU per ml is used.

As used herein, "carbon end-product" means any carbon product produced from the carbon intermediate, wherein the substrate contains at least one carbon atom (*i.e*., a carbon substrate).

As used herein, "carbon intermediate" refers to the carbon-containing compounds that are produced during the conversion of a carbon-containing substrate to a carbon end-product.

As used herein, " enzymatically controlled" means regulating the amount of carbon intermediate produced from the carbon substrate by altering the amount or activity of the enzyme used in the reaction.

As used herein, "microorganism" refers to any organism with cells that are typically considered to be microscopic, including such organisms as bacteria, fungi (yeasts and molds), rickettsia, and protozoa. It is not intended that the present invention be limited to any particular microorganism(s) or species of microorganism(s), as various microorganisms and microbial enzymes are suitable for use in the present invention. It is also not intended that the present invention be limited to wild-type microorganisms, as microorganisms and microbial enzymes produced using recombinant DNA technologies also find use in the present invention.

As used herein, "microbial enzyme" refers to any enzyme that is produced by a microorganism. As used herein, a "microbial intermediate-converting enzyme" is an enzyme that converts an intermediate to an end-product, while a "microbial substrate-converting enzyme" is an enzyme that converts a substrate to an intermediate or directly converts a substrate to an end-product (*i.e.*, there is not intermediate compound).

As used herein, "gluconic acid" refers to an oxidative product of glucose, wherein the C6 hydrozyl group of glucose is oxidized to a carboxylic acid group.

As used herein, the terms "gluconic acid producer" and "gluconic acid producing organism" refers to any organism or cell that is capable of producing gluconic acid through the use of a hexose or a pentose. In some embodiments, gluconic acid producing cells contain a cellulase as a substrate converting enzyme, and glucose oxidase and catalase for the conversion of the intermediates to gluconic acid.

As used herein, "glycerol producer" and "glycerol producing organism" refer to any organism or cell capable of producing glycerol. In some embodiments, glycerol producing organisms are aerobic bacteria, while in other embodiments, they are anaerobic bacteria. In still further embodiments, glycerol producing organisms include microorganisms such as fungi (*i.e*., molds and yeast), algae and other suitable organisms.

As used herein, the terms "diol producer," "propanediol producer," "diol producing organism," and "propanediol producing organism" refer to any organism that is capable of producing 1,3-propanediol utilizing glycerol. Generally, diol producing cells contain either a diol dehydratase enzyme or a glycerol dehydratase enzyme.

As used herein, the terms "lactate producer," and "lactate producing organism," and "lactate producing microorganism" refer to any organism or cell that is capable of producing lactate by utilizing a hexose or a pentose. In some embodiments, the lactate producers are members of the genera *Lactobacillus* or *Zymomonas,* while in other embodiments, they organisms are fungi.

As used herein, the terms "ethanol producer" and "ethanol producing organism" refer to any organism or cell that is capable of producing ethanol from a hexose or a pentose. Generally, ethanol producing cells contain an alcohol dehydrogenase and pyruvate decarboxylase.

As used herein, the term "ascorbic acid intermediate producer" and "ascorbic acid intermediate producing organism" refers to any organism or cell that is capable of producing an ascorbic acid intermediate from a hexose or a pentose. Generally, ethanol producing cells contain a glucose dehydrogenase, gluconic acid dehydrogenase, 2,5-diketo-D-gluconate reductase, 2-keto-D-gluconate reductase, 2-keto-reductase, 5-keto reductase, glucokinase, glucono kinase, ribulose-5-phosphate epimerase, transketolase, transaldolase, hexokinase, 2,5-DKG reductase, and/or idonate dehydrogenase, depending upon the specific ascorbic acid intermediate desired.

As used herein, the term "ascorbic acid intermediate intermediate" refers to any of the following compounds: D-gluconate, 2-keto-D-gluconate (2KDG), 2,5-diketo-D-gluconate (2,5-DKG or5 DKG), 2-keto-L-gulonic acid (2KLG or KLG), L-idonic acid (IA), erythorbic acid (EA), and ascorbic acid (ASA).

As used herein, "citric acid" refers to having the formula C₆H₈O₇, commonly found in citrus fruits, beets, cranberries and other acid fruits. The term refers to citric acid from any source, whether natural or synthetic, as well as salts and any other form of the acids.

As used herein, "succinic acid" refers to the acid having the formula C₄H₆O₄, which is commonly found in amber, algae, lichens, sugar cane, beets and other plants. This acid is also formed during the fermentation of sugar, tartrates, malates, and other substances by various molds, yeasts and bacteria. The term refers to succinic acid from any source, whether natural or synthetic, as well as acid and neutral salts and esters, and any other form of the acid.

As used herein, "amino acid" refers to any of naturally-occurring amino acids, as well as any synthetic amino acids, including amino acid derivatives.

As used herein, "antimicrobial" refers to any compound that kills or inhibits the growth of microorganisms (including but not limited to antibacterial compounds).

As used herein, the term "linked culture" refers to a fermentation system that employs at least two cell cultures, in which the cultures are added sequentially. In most embodiments of linked systems, a primary culture or a set of primary cultures is grown under optimal fermentation conditions for the production of a desired intermediate (*i.e*., the intermediate is released into the culture media to produce a "conditioned medium"). Following the fermentation of the primary culture, the conditioned medium is then exposed to the secondary culture(s). The secondary cultures then convert the intermediate in the conditioned media to the desired end-product. In some embodiments of the present invention, the primary cultures are typically glycerol producers and the secondary cultures are 1,3-propanediol producers.

As used herein, "mixed culture" refers to the presence of any combination of microbial species in a culture. In some preferred embodiments, the mixed culture is grown in a reaction vessel under conditions such that the interaction of the individual metabolic processes of the combined organisms results in a product which neither individual organism is capable of producing. It is not intended that the present invention be limited to mixed cultures comprising a particular number of microbial species.

As used herein, "conditioned media" refers to any fermentation media suitable for the growth of microorganisms that has been supplemented by organic by-products of microbial growth. In preferred embodiments of the present invention, conditioned media are produced during fermentation of linked cultures wherein glycerol producing cells secrete glycerol into the fermentation media for subsequent conversion to 1,3-propanediol.

As used herein, "oxygen uptake rate" ("OUR") refers to the determination of the specific consumption of oxygen within a reaction vessel. Oxygen consumption can be determined using various on-line measurements known in the art. In one embodiment, the OUR (mmol/(liter*hour)) is determined by the following formula: ((Airflow (standing liters per minute) / Fermentation weight (weight of the fermentation broth in kilograms)) X supply O₂ X broth density X (a constant to correct for airflow calibration at 21.1 C instead of standard 20.0 C)) minus ([airflow /fermentation weight] x [offgas O₂/offgas N₂] X supply N₂ X broth density X constant ).

As used herein, "carbon evolution rate" ("CER") refers to the determination of how much CO₂ is produced within a reaction vessel during fermentation. Usually, since no CO₂ is initially or subsequently provided to the reaction vessel, any CO₂ is assumed to be produced by the fermentation process occurring within the reaction vessel. "Off-gas CO₂" refers to the amount of CO₂ measured within a reaction vessel, usually by mass spectroscopic methods known in the art.

As used herein, the term "enhanced" refers to improved production of proteins of interest. In preferred embodiments, the present Invention provides enhanced (*i.e.*, improved) production and secretion of a protein of interest. In these embodiments, the "enhanced" production is improved as compared to the normal levels of production by the host (*e.g*., wild-type cells). Thus, for heterologous proteins, basically any expression is enhanced, as the cells normally do not produce the protein.

As used herein, the terms "isolated" and "purified" refer to a nucleic acid or amino acid that is removed from at least one component with which it is naturally associated.

As used herein, the term "heterologous protein" refers to a protein or polypeptide that does not naturally occur in a host cell. In alternate embodiments, the protein is a commercially important industrial protein or peptide. It is intended that the term encompass protein that are encoded by naturally occurring genes, mutated genes, and/or synthetic genes.

### Substrates

During the development of the present invention good results were obtained with corn starch and wheat starch, although other sources, including starches from grains and tubers (*e.g*., sweet potato , potato , rice and cassava starch) also find use with the present invention. Various starches are commercially available. For example, corn starches are available from Cerestar, Sigma, and Katayama Chemical Industry Co. (Japan); wheat starches are available from Sigma; sweet potato starch is available from Wako Pure Chemical Industry Co. (Japan); and potato starch is available from Nakari Chemical Pharmaceutical Co. (Japan). A particularly useful carbon substrate is corn starch. In some embodiments of the present invention, granular starch is used in a slurry having a percentage of starch between about 20% and about 35%. Preferably, the starch is in a concentration between about 10% and about 35%. In some particularly preferred embodiments, the range for percent starch is between 30% and 32%.

### Enzymes

The alpha-amylase used in the present invention is generally an enzyme which effects random cleavage of alpha-(1-4) glucosidic linkages in starch. In most embodiments, the alpha-amylase is chosen from among the microbial enzymes having an E. C. number E. C. 3.2.1.1 and in particular E. C. 3.2.1.1-3. In some preferred embodiments, the alpha-amylase is a thermostable bacterial alpha-amylase. In most particularly preferred embodiments, the alpha-amylase is obtained or derived from *Bacillus* species. Indeed, during the development of the present invention good results were obtained using the SPEZYME® alpha-amylase obtained from *Bacillus licheniformis* (Genencor). In other embodiments, black-koji amylase described in methods for alcoholic fermentation from starch such as corn and cassava without precooking (Ueda et al., J. Ferment. Technol., 50:237-242 [1980]; and Ueda et al, J. Ferment. Technol., 58:237-242 [1980]) find use in the present invention.

As understood by those in the art, the quantity of alpha-amylase used in the methods of the present invention will depend on the enzymatic activity of the alpha-amylase and the rate of conversion of the generated glucose by the end-product converter. For example, generally an amount between 0.01 and 1.0 kg of SPEZYME® FRED (Genencor) is added to one metric ton of starch. In some embodiments, the enzyme is added in an amount between 0.4 to 0.6 kg, while in other embodiments, it is added in an amount between 0.5 and 0.6 kg of SPEZYME® FRED/metric ton of starch..

In preferred embodiments of the present invention, the glucoamylase is an enzyme which removes successive glucose units from the non-reducing ends of starch. The enzyme can hydrolyze both the linear and branched glucosidic linkages of starch, amylose and amylopectin. In most embodiments, the glucoamylase used in the methods of the present invention are microbial enzymes. In some preferred embodiments, the glucoamylase is a thermostable fungal glucoamylase, such as the *Aspergillus* glucoamylase. Indeed, during the development of the present invention, good results were obtained using the DISTALLASE® glucoamylase derived from *Aspergillus niger* (Genencor ). Glucoamylase preparations from *Aspergillus niger* have also been used without the use of precooking (*See*, Ueda et al, Biotechnol. Bioeng., 23:291 [1981]). Three glucoamylases have been selectively separated from *Aspergillus awamori var. kawachi* for use in hydrolyzing starch (*See*, Hayashida, Agr. Biol. Chem., 39:2093-2099 [1973]). Alcoholic fermentation of sweet potato by *Endomycopsis fibuligoeu* glucoamylase without cooking has also been described (Saha et al., Biotechnol. Bioeng., 25:1181-1186 [1983]). Another enzyme that finds use in the present invention is glucoamylase (EC 3.2.1.3), an enzyme that hydrolyzes the alpha.-1,4-glucoside chain progressively from the non-reducing terminal end. This enzyme also hydrolyzes the alpha-1,6-glucoside chain. Glucoamylase is secreted from fungi of the genera *Aspergillus, Rhizopus* and *Mucor* also find use in the methods of the present invention. These enzymes further find use in glucose production and quantitative determination of glycogen and starch. Glucoamylase preparations obtained from *E. fibuligera* (IFO 0111) have been used to contact raw sweet potato starch for alcoholic fermentation (*See*, Saha et al., Biotechnol. Bioeng., 25:1181-1186 [1983]). One of this enzyme's major applications is as a saccharifying agent in the production of ethyl alcohol from starchy materials. However, as with the other glucoamylases described herein, this enzyme also finds use in the methods of the present invention.

Additional glucoamylases that find use in the methods of the present invention include those obtained from the genera *Rhizopus and Humicola,* which are characterized as having particularly high productivity and enzymatic activity. Furthermore, in comparison with the glucoamylase derived from other organisms, the *Rhizopus*-derived glucoamylase exhibits a strong action on starch and its enzymological and chemical properties including optimum pH are particularly suitable for the saccharification of cereal starch. Because of these features, the Rhizopus-derived glucoamylase is considered to be best suited for alcohol production using non-cooked or low-temperature cooked starch (*See*, U.S. Pat. No. 4,514,496 and 4,092,434). It has been noted that upon the incubation of raw corn starch with *Rhizopus* glucoamylase, was used in conjunction with *Rhizopus* alpha amylase, the starch degradation by glucoamylase was accelerated. While it is not intended that the present invention be limited to any particular mechanism or theory, it is believed that *Rhizopus* glucoamylase has a stronger degradation activity than *Aspergillus niger* glucoamylase preparations which also contain α-amylase (*See*, Yamamoto et al., Denpun Kagaku, 37:129-136 [1990]). One commercial preparation that finds use in the present invention is the glucoamylase preparation derived from the Koji culture of a strain of *Rhizopus niveus* available from Shin Nippo Chemical Co., Ltd. Another commercial preparation that finds use in the present invention is the commercial starch hydrolyzing composition M1 is available from Biocon India, of Bangalore, India.

As understood by those in the art, the quantity of glucoamylase used in the methods of the present invention depends on the enzymatic activity of the glucoamylase (*e.g*., DISTILLASE® L-400). Generally, an amount between 0.001 and 2.0 ml of a solution of the glucoamylase is added to 450 gm of a slurry adjusted to 20-35% dry solids, the slurry being the liquefied mash during the saccharification and/or in the hydrolyzed starch and sugars during the fermentation. In some embodiments, the glucoamylase is added in an amount between 0.005 and 1.5 ml of such a solution. In some preferred embodiments, the enzyme is added at an amount between 0.01 and 1.0 ml of such a solution.

As indicated above, pullulanases also find use in the methods of the present invention. These enzymes hydrolyze alpha.-1,6-glucosidic bonds. Thus, during the saccharification of the liquefied starch, pullulanases remove successive glucose units from the non-reducing ends of the starch. This enzyme is capable of hydrolyzing both the linear and branched glucosidic linkages of starch, amylose and amylopectin.

Additional enzymes that find use in the present invention include starch hydrolyzing (RSH) enzymes, including *Humicola* RSH glucoamylase enzyme preparation (*See*, U.S. Patent No. 4,618,579). This *Humicola* RSH enzyme preparation exhibits maximum activity within the pH range of 5.0 to 7.0 and particularly in the range of 5.5 to 6.0. In addition, this enzyme preparation exhibits maximum activity in the temperature range of 50° C. to 60° C. Thus, in each of the steps of the present invention in which this enzyme is used, the enzymatic solubilization of starch is preferably carried out within these pH and temperature ranges.

In some embodiments, *Humicola* RSH enzyme preparations obtained from the fungal organism strain *Humicola grisea var. thermoidea* find use in the methods of the present invention. In some particularly preferred embodiments, these *Humicola* RSH enzymes are selected from the group consisting of ATCC (American Type Culture Collection) 16453, NRRL (USDA Northern Regional Research Laboratory) 15219, NRRL 15220, NRRL 15221, NRRL 15222, NRRL 15223, NRRL 15224, and NRRL 15225, as well as genetically altered strains derived from these enzymes.

Additional RSH glucoamylases that find use in the methods of the present invention include *Rhizopus* RSH glucoamylase enzyme preparations. In some embodiments, the enzyme obtained from the Koji strain of *Rhizopus niveus* available from Shin Nihon Chemical Co., Ltd., Ahjyo, Japan, under the tradename "CU CONC" is used. Another useful enzyme preparation is a commercial digestive from *Rhizopus* available from Amano Pharmaceutical under the tradename "GLUCZYME" (*See*, Takahashi et al., J. Biochem., 98:663-671 [1985]). Additional enzymes include three forms of glucoamylase (EC 3.2.1.3) of a *Rhizopus* sp., namely "Gluc1" (MW 74,000), "Gluc2" (MW 58,600) and "Gluc 3" (MW 61,400). Gluc1 was found to be 22-25 times more effective than Gluc2 or Gluc3. Thus, although Gluc2 and Gluc3 find use in the present invention, because Gluc1 tightly binds to starch and has an optimum pH of 4.5, Gluc1 finds particular use in the present invention. An additional RSH glucoamylase enzyme preparation for use in the present invention includes enzyme preparations sold under the designation "M1," available from Biocon India, Ltd., Bangalore, India (M1 is a multifaceted enzyme composition or mixture).

As noted above, in most embodiments, *Humicola* RSH glucoamylase enzyme preparations contain glucoamylase activity as well as a potentiating factor which solubilizes starch. The relative proportions of potentiating factor and glucoamylase activity in other RSH enzyme preparations may vary somewhat. However, with RSH glucoamylase enzyme preparations that find use in the present invention, there is usually ample potentiating factor produced along with the glucoamylase fraction. Accordingly, the activity of the RSH glucoamylase enzyme preparations is defined in terms of their glucoamylase activity.

*Lactobacillus amylovorous* (ATCC 33621) is a lactic acid producing bacteria isolated from cattle manure corn enrichments (*See*, Nkamura, Int. J. Syst. Bacteriol., 31:56-63 [1981]). This strain produces an extracellular amylase which enables it to hydrolyze liquefied (soluble) starch to glucose, which can then be fermented to lactic acid. (*See*, Xiaodong et al., Biotechnol. Lett., 19:841-843 [1997]).

Indeed, commercially available alpha-amylases and glucoamylases find use in the methods of the present invention in economically realistic enzyme concentrations. Although commonly used fermentation conditions do not utilize optimum temperatures, the pH conditions for fermentation do correspond closely to the optimum pH for commercially available saccharification enzymes (*i.e*., the glucoamylases). In some embodiments of the present invention, complete saccharification to glucose is favored by the gradual solubilization of granular starch. Presumably, the enzyme is always exposed to low concentrations of dextrin. In addition, the removal of glucose throughout the fermentation maintains a low glucose content in the fermentation medium. Thus, glucoamylase is exposed to low concentration of glucose. In consequence, the glucoamylase is used so effectively that economically feasible dosage levels of glucoamylase (GAU) are suitable for use in the methods of the present invention (*i.e*., glucoamylase dosage of 0.05-10.0 GAU/g of starch; and preferably 0.2-2.0 GAU/g starch).

The dosages provided above for glucoamylase only approximate the effective concentration of the enzymatic saccharification activity in the fermentation broth, as an additional proportion of the saccharification activity is contributed by the alpha-amylase. Although it is not intended that the present invention be limited to any particular mechanism or theory, it is believed that the alpha-amylase further widens the holes bored by glucoamylase on starch granules (*See*, Yamamoto *et al., supra*). Typically, the use of commercially available alpha-amylases results in the production of significant amounts of sugars, such as glucose and maltose.

Furthermore, some commercially available glucoamylases contain some alpha-amylase activity. Thus, it is possible (albeit usually not practical) to ferment particulate starch in the presence solely of glucoamylase. However, it is not intended that such embodiments be excluded from the present invention.

In most embodiments of the methods of the present invention, an effective amount of alpha-amylase is added to a slurry of particulate starch. Those of skill in the art understand that in addition to the uncertain amount of alpha-amylase activity contributed by glucoamylase, the effective activity of the alpha-amylase may be quite different from the unit activity values given by the supplier. The activity of alpha-amylase is pH dependent, and may be different at the pH range selected for the fermentation (*i.e.*, as compared with the test conditions employed by the suppliers for their reported unit activity values). Thus, some preliminary experiments are contemplated as being sometimes necessary in order establish the most effective dosages for alpha-amylases, including those not explicitly described herein, but find use in the methods of the present invention.

In some most preferred embodiments, the alpha-amylase dosage range for fungal alpha-amylases is from 0.02 SKBU/g (Fungal Alpha Amylase Units) to 2.0 SKBU/g of starch, although in some particularly preferred embodiments, the range is 0.05-0.6 SKBU/g. One "SKBU" is as known in the art (*See*, Cerial Chem., 16:712-723 [1939]). In most embodiments utilizing *Bacillus* alpha-amylases, the range is 0.01 LU/g to 0.6 LU/g, preferably 0.05 to 0.15 LU/g. It is contemplated that the uncertainty as to the real activity of both the glucoamylase and the alpha-amylase in the fermenting slurry will require some preliminary investigation into the practice of some embodiments. Optimization considerations include the fact that increasing the alpha-amylase dosage with a constant glucoamylase content, increases the fermentation rate. In addition, increasing the glucoamylase dosage with a constant alpha-amylase content increases the fermentation rate. Holding the dosage of enzyme constant and/or increasing the starch content in the slurry also increase the fermentation rate. Indeed, it is contemplated that in some embodiments, the optimum alpha-amylase dosage well exceeds dosages heretofore recommended for liquefying starch; the optimum glucoamylase may well exceed dosages recommended for saccharifying syrups. However, enzyme dosage levels should not be confused with enzyme usage. Substantial proportions of the enzymes dosed into the starch scurry are available for recovery from the fermentation broth for use anew to ferment granular starch.

A further consideration arising from employment of the enzymes at fermentation temperatures is that although the enzymes exhibit low relative activity (*e.g*., activity of the alpha-amylase from *B. licheniformis* at fermentation temperatures is not more than about 25% of maximum activity), the low relative activity is counterbalanced by the extended duration of the 48-120 hours of fermentation, and by the extended half-life of enzymes that have not been subjected to elevated temperatures. Indeed, it has been determined that more than 90% of the enzymes activity remains after 72 hours of fermentation.

The alpha-amylase of *B. licheniformis* (SPEZYME® AA and SPEZYME® FRED enzymes; Genencor International Inc.) is sufficiently stable to withstand brief exposures to still pot temperatures. Thus, recycle of stillage can be used as a way to recycle alpha-amylase.

However, as earlier described, some RSHs glucoamylases (e.g., the enzyme obtained from *Rhizopus*) are available that convert starch to glucose at non-cooking temperatures, reducing the need for exposing the enzymatic composition to still pot temperatures. This reduces the energy costs of converting the carbon substrate to the desired end-product, thereby reducing the overall costs of manufacturing. Thus, these enzymes find particular use in the methods of the present invention.

### Culture Conditions

Typically, cells are grown at approximately 30 °C. in appropriate media. Preferred growth media utilized in the present invention include common commercially prepared media such as Luria Bertani (LB) broth, Sabouraud Dextrose (SD) broth or Yeast Malt Extract (YM) broth. However, other defined or synthetic growth media may also be used, as appropriate. Appropriate culture conditions are well-known to those in the art.

In some embodiments, agents known to modulate catabolite repression directly or indirectly (*e.g*., cyclic adenosine 2':3'-monophosphate or cyclic adenosine 2':5'-monophosphate), are incorporated into the reaction media. Similarly, the use of agents known to modulate enzymatic activities (*e.g*., sulphites, bisulphites and alkalis) that lead to enhancement of glycerol production also find use in conjunction with or as an alternative to genetic manipulations.
Suitable pH ranges for fermentation are between pH 5.0 to pH 9.0; while the range of pH 6.0 to pH 8.0 is particularly preferred for the initial conditions of the reaction system. Furthermore, reactions may be performed under aerobic, microaerophilic, or anaerobic conditions, as suited for the organism utilized.

### Batch and Continuous Fermentations

In some preferred embodiments, the present process uses a batch method of fermentation. A classical batch fermentation is a closed system, wherein the composition of the media is set at the beginning of the fermentation and is not subject to artificial alterations during the fermentation. Thus, at the beginning of the fermentation the medium is inoculated with the desired organism(s). In this method, fermentation is permitted to occur without the addition of any components to the system. Typically, a batch fermentation qualifies as a "batch" with respect to the addition of the carbon source and attempts are often made at controlling factors such as pH and oxygen concentration. The metabolite and biomass compositions of the batch system change constantly up to the time the fermentation is stopped. Within batch cultures, cells moderate through a static lag phase to a high growth log phase and finally to a stationary phase where growth rate is diminished or halted. If untreated, cells in the stationary phase eventually die. In general, cells in log phase are responsible for the bulk of production of end product or intermediate.

A variation on the standard batch system is the "fed-batch fermentation" system, which also finds use with the present invention. In this variation of a typical batch system, the substrate is added in increments as the fermentation progresses. Fed-batch systems are useful when catabolite repression is apt to inhibit the metabolism of the cells and where it is desirable to have limited amounts of substrate in the media. Measurement of the actual substrate concentration in fed-batch systems is difficult and is therefore estimated on the basis of the changes of measurable factors such as pH, dissolved oxygen and the partial pressure of waste gases such as CO₂. Batch and fed-batch fermentations are common and well known in the art.

It is also contemplated that the methods of the present invention are adaptable to continuous fermentation methods. Continuous fermentation is an open system where a defined fermentation media is added continuously to a bioreactor and an equal amount of conditioned media is removed simultaneously for processing. Continuous fermentation generally maintains the cultures at a constant high density where cells are primarily in log phase growth.

Continuous fermentation allows for the modulation of one factor or any number of factors that affect cell growth and/or end product concentration. For example, in one embodiment, a limiting nutrient such as the carbon source or nitrogen level is maintained at a fixed rate an all other parameters are allowed to moderate. In other systems, a number of factors affecting growth can be altered continuously while the cell concentration, measured by media turbidity, is kept constant. Continuous systems strive to maintain steady state growth conditions. Thus, cell loss due to media being drawn off must be balanced against the cell growth rate in the fermentation. Methods of modulating nutrients and growth factors for continuous fermentation processes as well as techniques for maximizing the rate of product formation are well known in the art of industrial microbiology.

In some embodiments, the present invention is practiced using batch processes, while in other embodiments, fed-batch or continuous processes, as well as any other suitable m0de of fermentation are used. Additionally, in some embodiments, cells are immobilized on a substrate as whole-cell catalysts and are subjected to fermentation conditions for the appropriate end-product production.

### Identification and Purification of the End-Product

Methods for the purification of the end-product from fermentation media are known in the art.

In some embodiments, the end-product is identified directly by submitting the media to high pressure liquid chromatography (HPLC) analysis. One method of the present invention involves analysis of fermentation media on an analytical ion exchange column using a mobile phase of 0.01 N sulfuric acid in an isocratic fashion.

### Process Considerations

As indicated above, fermentation of granular starch slurry has completely different characteristics than fermentation of a syrup. Generally, a concentration of about 20% solids in solution is considered the maximum sugar content in a fermentation medium, as higher concentrations create difficulties at the onset and at the end of fermentation. However, no similar limits exist in the fermentation of a starch slurry. The concentration of starch in the slurry may vary from 10 -35 %, with no discernable consequences at the onset of fermentation. Increasing starch concentration (*e.g*., at constant enzyme dosages) speeds up the bioconversion rate, or conversely, allows for lowering the enzyme dosages required to achieve a given bioconversion rate. The excess (*i.e*., residual) granular starch may be recovered, along with substantial amounts of enzymes and subjected to renewed fermentation. Thus, control over starch concentration is a major process parameter for practice of this invention.

In one preferred embodiment, means for bioconversion and fermentation of a granular starch slurry having 10-35% starch by weight are provided. The reaction is dependent on the microorganism and bioprocessing conditions used and, therefore, recycling of the enzymes on the starch particles occurs when the residual starch is again fermented. However, even when a 25-35% starch slurry is fermented, in preferred embodiments, the fermentation is halted before complete disappearance of the granular starch, for fermentation anew. Thus, recycling of starch is a facile way to recover enzymes for reuse.

In one preferred embodiment of the present invention, the (granular) starch and microorganisms are removed together (*e.g*., by centrifugation or filtration). This removed starch and microorganisms are mixed with fresh granular starch and additional aliquot(s) of enzyme(s) as needed, to produce a fermentation charge for another fermentation run.

As recognized by those of skill in the art, engineering trade-offs are contemplated in arriving at optimum process details; these trade-offs are contemplated to vary, depending upon each particular situation. Nonetheless, the methods provided herein provide the necessary teachings to make such trade-offs to obtain optimum processes. For example, to achieve the most rapid fermentation reasonable, high starch content, and high enzymes dosage are indicated. But, the consequential rapid fermentation tails off into generation of a level of nutrients in the fermentation broth, when then dictates recovery of the nutrients, or, alternatively that fermentation be halted at a relatively low end-product content. However, in situations where relatively low fermentation rates are acceptable, then (with high starch content slurries) enzyme dosage is relatively low and nutrient losses are held to levels heretofore accepted by the fermentation arts. In cases where maximum yield of end-product is a principal objective, then low starch content slurries, moderate alpha-amylase dosage, and high glucoamylase dosage find use in the present invention. However, it is not intended that the present invention be limited to any particular method design.

As indicated herein, the present invention saves considerable thermal energy. However, just as the starting starch substrate is never subjected to the thermal conditions used for liquefactions, the substrate is not thermally sterilized. Thus, it is contemplated that in some embodiments, the granular starch substrate may add contaminating microorganisms to the fermentation medium. Thus, in some embodiments, it is advantageous to seed the fermentation medium with a large number of the product-producing microorganisms that are associated with recycled substrate (*e.g*., starch). By greatly outnumbering the contaminants, the recycled microorganisms overwhelm any contaminating microorganisms, thereby dominating the fermentation, resulting in the production of the desired end-product.

In some embodiments, the quantities of microorganisms and/or enzymes initially charged into the fermentation vat or bioreactor are in accord with prior art practices for the fermentation and/or bioconversion of various products. These quantities will vary, as the microbial cells multiply during the course of the fermentation whereas enzymes used for bioconversion will have a limited half-life. Although in some embodiments, recycling of microorganisms is utilized, in many embodiments, it is not required for the practice of the present invention. In contrast, in particularly preferred embodiments, it is desirable to recycle enzymes (although it is not intended that the present invention be limited to methods which require the recycling of enzymes).

Thus, in some embodiments, removal of the microbes from the residual starch particles prior to recycling of the residual starch is contemplated. However, it is again noted that practice of the present invention does not necessarily require thermal treatment of the starting substrate, Thus, in some embodiments, the starting substrate is heat-sterilized, while in other embodiments, it is not. Therefore, in some embodiments, the fermentation/bioconversion is conducted in the presence of a relatively large proportion of microorganisms, in order to overcome the effects of any contamination. In alternative embodiments, antimicrobials are added to the fermentation medium to suppress growth of contaminating microorganisms. In additional embodiments, cold sterilization techniques, UV radiation, 65°C pasteurization are used to sterilize the starting (*e.g*,, substrate) materials. However, biomass poses no problem regarding sterilization of fermentation vats or bioreactors.

As described herein, the present invention provides means to control the fermentation rate by releasing metabolizable sugars to the microbes at a controlled rate. The methods of the present invention are very different from what has been done heretofore. The prior art teaches the treatment of solid starch with enzymes prior to fermentation and/or inclusion of enzymes in the fermentation medium to conserve energy and/or to improve fermentation efficiency. However, in contrast to the present invention, there is no teaching in the art to alter the character of the fermentation so as to achieve a near to linear fermentation rate. The present invention provides means to efficiently conserve energy, particularly as compared to high temperature starch liquefaction. Indeed, in preferred embodiments, more thermal energy is conserved. The methods of the present invention operate with high fermentation efficiency, in part because product losses due to starch retrogradation, incomplete saccharification, and incomplete fermentation of fermentables are reduced. Furthermore, the ability to tailor the fermentation rate through control of starch or biomass concentration, as well as controlling the enzyme content and proportions, as provided by the present invention, facilitates the production of the desired end-products with minimal carbohydrate content.

### EXPERIMENTAL

The following examples are provided in order to demonstrate and further illustrate certain preferred embodiments and aspects of the present invention and are not to be construed as limiting the scope thereof. Indeed, it is contemplated that these teachings will find use in further optimizing the process systems described herein.

In the experimental disclosure which follows, the following abbreviations apply: wt% (weight percent); °C (degrees Centigrade); rpm (revolutions per minute); H₂O (water); dH₂O (deionized water); (HCl (hydrochloric acid); aa (amino acid); bp (base pair); kb (kilobase pair); kD (kilodaltons); gm (grams); µg (micrograms); mg (milligrams); ng (nanograms); µl (microliters); ml and mL (milliliters); mm (millimeters); nm (nanometers); µm (micrometer); M (molar); mM (millimolar); µM (micromolar); U (units); V (volts); MW (molecular weight); psi (pounds per square inch); sec (seconds); min(s) (minute/minutes); hr(s) (hour/hours); Q.S. and q.s. (quantity sufficient); OD (optical density); OD₂₈₀ (optimal density at 280 nm); OD₆₀₀ (optical density at 600 nm); PAGE (polyacrylamide gel electrophoresis); DO (dissolved oxygen); Di (deionized); phthalate buffer (sodium phthalate in water, 20 mM, pH 5.0); PBS (phosphate buffered saline [150 mM NaCl, 10 mM sodium phosphate buffer, pH 7.2]); Cerestar granular starch (Cargill Foods PFP2200 granular starch; ); Cerestar (Cerestar, Inc., a Cargill Inc., company, Minneapolis, MN); AVICELL®(FMC Corporation); SDS (sodium dodecyl sulfate); Tris (tris(hydroxymethyl)aminomethane); w/v (weight to volume); v/v (volume to volume); slpm (standardized liters per minute); ATCC (American Type Culture Collection, Rockville, MD); Difco (Difco Laboratories, Detroit, MI); GIBCO BRL or Gibco BRL (Life Technologies, Inc., Gaithersburg, MD); Genencor (Genencor International, Inc., Palo Alto, CA); Shin Nihon (Shin Nihon, Japan).

In the following example and related work, additional various media and buffers known to those in the art were used, including the following:
*Lactobacilli* MRS Media (for inoculum): Difco (Ref# 288130):
0.5x Modified *Lactobacilli* MRS Media w/o glucose + 8% granular starch recipe:

| | |
|---|---|
| Yeast extract (Difco) | 15.0 g/L |
| Granular starch (Cerestar) | 80.0 g/L |
| MgSO₄*7H₂O | 0.3 g/L |
| KH₂PO₄ | 0.5 g/L |
| K₂HPO₄ | 0.5 g/L |
| Sodium acetate | 0.5 g/L |
| FeSO₄*7H₂O | 0.03 g/L |
| MnSO₄*1H₂O | 0.03 g/L |
| Mazu DF204 (antifoam) | 1ml |
| 1000x Tiger trace metal | 0.2m/s stock solution |

TM2 Recipe (per liter fermentation medium):
K₂HPO₄ 13.6 g, KH₂PO₄ 13.6 g, MgSO₄ * 7H₂O 2 g, citric acid monohydrate 2 g, ferric ammonium citrate .3 g, (NH₄)₂SO₄ 3.2 g, yeast extract 5 g, 1000X Modified Tiger Trace Metal Solution 1 ml. All of the components are added together and dissolved in diH₂O. The pH is adjusted to 6.8 with potassium hydroxide (KOH) and q.s. to volume. The final product is filter sterilized with 0.22 u (micron) filter (only do not autoclave).
Murphy III Medium (g/l)
KH₂PO₄ (12g), K₂HPO₄ (4 g), MgSO₄.7H₂O (2 g), DIFCO Soytone (2 g), sodium citrate (0.1 g), fructose (5 g), (NH₄)₂SO₄ (1 g), nicotinic acid (0.02 g), 0.4 g/l FeCl₃.6H2O (5 ml), and Pho salts (5 ml).
1000X Modified Tiger Trace Metal Solution:
Citric Acids * H₂O 40 g, MnSO₄ * H₂O 30 g, NaCl 10 g, FeSO₄ * 7H₂O 1 g, CoCl₂ * 6H₂O 1 g, ZnSO * 7H₂O 1 g, CuSO₄ * 5H₂O 100 mg, H₃BO₃ 100 mg, NaMoO₄ * 2H₂O 100 mg. Each component is dissolved one at a time in Di H₂O, pH to 3.0 with HCl/NaOH, then q.s. to volume and filter sterilize with 0.22 micron filter.

### EXAMPLE 1

### Conversion of Starch to Lactic Acid

This experiment was carried out in 1 L bioreactor to monitor lactate formation from granular starch using enzymes with glucoamylase activity at desired fermentation pH 6.4 and temperature 34°C. In this experiment, granular starch in slurry form (maximum final concentration of 80 g/L glucose) in the 0.5x modified *Lactobacilli* medium fermentation medium, was pasteurized (*i.e.*, the mixture was held at 34°C for 30 min for germination of any contaminant present in the starch slurry, and then pasteurized at 65 °C for 14 hr). This was added to the pre-sterilized 1L bioreactor. The pH of the slurry/broth was adjusted to 6.4 and controlled at 6.4 with 28% NH₄OH. Then, the desired enzymes (0.4g of sumizyme CU CONC™; Shin Nihon) were added as 0.2 micron filtered solution (20ml) in DI water. Then, an inoculum of lactate-producing strain *Lactobacillus casei* (ATCC 393), taken from a frozen vial, was prepared in *Lactobacillus* MRS medium (Difco). After the inoculum grew to OD 2.4, measured at 550 nm, in a 1 L bioreactor at 34°C with a nitrogen sparge at 0.6 slpm (standardized liters per minute) flow rate), the contents of the reactor (600ml) were centrifuged and re-suspended in 45ml supernatant to transfer the cell pellet (42ml of OD22 material) as the inoculum for the fermentative bioconversion in a bioreactor. For the duration of the fermentative bioconversion run, nitrogen was sparged at 0.6 slpm, the back pressure was held at 5psi, the temperature was held at 34°C, pH held at 6.4 by base titration of 28% NH₄OH.

During the reaction, samples were taken from the vessel, centrifuged, and the supernatants were refrigerated to terminate the enzyme action. The supernatant was then subjected to HPLC analysis. This experiment monitored bioconversion of granular starch by measuring glucose formation and its conversion to lactate. In 16.3 hours, accumulation of lactate amounted to 61.75 g/L (Figure 1).

In addition, the bioconversion of granular starch to lactate was demonstrated to be at a level of 3.79 g/L-hour rate, at a temperature of 34 °C, and at pH 6.4.

## Claims

1. A method for producing lactic acid comprising:
contacting a slurry comprising granular starch with a glucoamylase which is capable of hydrolyzing both the linear and branched glucosidic linkages of starch, an alpha-amylase, and a lactate-producing Lactobacillus, in a simultaneous saccharification and fermentation process;
wherein said starch is not subjected to liquefaction or gelatinization.

2. The method of claim 1 wherein the granular starch is derived from wheat, barley, sweet potato, tapioca, corn, maize, cassava, milo, rye, bran or rice.

3. The method of claim 1, wherein the alpha-amylase is from Bacillus species.

4. The method of claim 1, wherein the alpha-amylase is from Bacillus licheniformis or Aspergillus oryzae.

5. The method of claim 1, wherein the glucoamylase is from Humicola or Rhizopus species.

6. The method of claim 1 wherein said Lactobacillus is Lactobacillus amylovorous.

7. The method of claim 1 wherein the glucoamylase and alpha-amylase are both from Rhizopus.

8. The method of claim 1, wherein the slurry comprising the granular starch is subject to 65°C pasteurization before contacting with the starch hydrolyzing enzyme.

9. The method of claim 1, wherein the pH of the medium is pH 5.0 to 9.0.

10. The method of claim 1, wherein the fermentation takes place at a temperature range of 25°C to 35°C.

11. The method of claim 1, wherein the granular starch in the slurry is in a concentration of 10% to 35% w/v.

12. The method of claim 1, further comprising recovering the lactic acid.

## Patentansprüche

1. Verfahren zur Herstellung von Milchsäure, umfassend:
das Kontaktieren einer Aufschlämmung, die granuläre Stärke umfasst, mit einer Glucoamylase, die in der Lage ist, sowohl die unverzweigten als auch die verzweigten Glucosidbindungen von Stärke zu hydrolysieren, einer α-Amylase und einem Lactat produzierenden Lactobacillus in einem gleichzeitigen Verzuckerungs- und Fermentationsvorgang;
worin die Stärke nicht Verflüssigung oder Gelierung unterzogen wird.

2. Verfahren nach Anspruch 1, worin die granuläre Stärke von Weizen, Gerste, Süßkartoffel, Tapioka, Korn, Kukuruz (Mais), Maniok, Milo, Roggen, Kleie oder Reis stammt.

3. Verfahren nach Anspruch 1, worin die α-Amylase aus Bacillus-Spezies stammt.

4. Verfahren nach Anspruch 1, worin die α-Amylase aus Bacillus licheniformis oder Aspergillus oryzae stammt.

5. Verfahren nach Anspruch 1, worin die Glucoamylase aus Humicola- oder Rhizopus-Spezies stammt.

6. Verfahren nach Anspruch 1, worin der Lactobacillus Lactobacillus amylovorus ist.

7. Verfahren nach Anspruch 1, worin die Glucoamylase und die α-Amylase beide von Rhizopus stammen.

8. Verfahren nach Anspruch 1, worin die Aufschlämmung, die die granuläre Stärke umfasst, bei 65 °C Pasteurisierung unterzogen wird, bevor sie mit dem Stärke hydrolyiserenden Enzym kontaktiert wird.

9. Verfahren nach Anspruch 1, worin der pH-Wert des Mediums 5,0 bis 9,0 beträgt.

10. Verfahren nach Anspruch 1, worin die Fermentation in einem Temperaturbereich von 25 °C bis 35 °C erfolgt.

11. Verfahren nach Anspruch 1, worin die granuläre Stärke in der Aufschlämmung in einer Konzentration von 10 % bis 35 % (Gew./Vol.) vorliegt.

12. Verfahren nach Anspruch 1, weiters umfassend das Gewinnen der Milchsäure.

## Revendications

1. Procédé de production d'acide lactique comprenant:
mettre en contact une boue comprenant un amidon granulaire avec une glucoamylase qui est apte à hydrolyser à la fois les liaisons glucosiques linéaires et branchées de l'amidon, une alpha-amylase et un Lactobacillus de production de lactate, dans un processus de saccharification et de fermentation simultanée;
où ledit amidon n'est pas soumis à une liquéfaction ou gélatinisation.

2. Procédé selon la revendication 1, dans lequel l'amidon granulaire est dérivé du blé, orge, patates douces, tapioca, grain, maïs, manioc, sorgho, seigle, son ou riz.

3. Procédé selon la revendication 1, dans lequel l'alpha-amylase est de l'espèce de Bacillus.

4. Procédé selon la revendication 1, dans lequel l'alpha-amylase est de Bacillus licheniformis ou Aspergillus oryzae.

5. Procédé selon la revendication 1, dans lequel la glucoamylase est des espèces Humicola ou Rhizopus.

6. Procédé selon la revendication 1, où ledit Lactobacillus est le Lactobacillus amylovorous.

7. Procédé selon la revendication 1, dans lequel la glucoamylase et alpha-amylase sont toutes les deux de Rhizopus.

8. Procédé selon la revendication 1, dans lequel la boue comprenant l'amidon granulaire est soumise à une pasteurisation à 65°C avant la mise en contact avec l'enzyme hydrolysant l'amidon.

9. Procédé selon la revendication 1, dans lequel le pH du milieu est le pH 5,0 à 9,0.

10. Procédé selon la revendication 1, dans lequel la fermentation a lieu à une plage de température de 25°C à 35°C.

11. Procédé selon la revendication 1, dans lequel l'amidon granulaire dans la boue est dans une concentration de 10% à 35% w/v.

12. Procédé selon la revendication 1, comprenant en outre la récupération de l'acide lactique.
